# EUROPEAN PATENT SPECIFICATION

(11) **EP 4 277 528 B1**
(45) Date of publication and mention of the grant of the patent: **20.11.2024**
(21) Application number: 22701730.8
(22) Date of filing: 11.01.2022
(51) Int. Cl.: A61B 5/248, A61B 5/00

(54) **DIAGNOSTIC SUPPORT APPARATUS, DIAGNOSTIC SUPPORT METHOD, AND DIAGNOSTIC SUPPORT PROGRAM**
DIAGNOSEUNTERSTÜTZUNGSVORRICHTUNG, DIAGNOSEUNTERSTÜTZUNGSVERFAHREN UND DIAGNOSEUNTERSTÜTZUNGSPROGRAMM
SYSTÈME D?AIDE AU DIAGNOSTIC, PROCÉDÉ D'AIDE AU DIAGNOSTIC, ET PROGRAMME D?AIDE AU DIAGNOSTIC

(30) Priority: 12.01.2021 JP 2021003138; 03.06.2021 JP 2021093758
(43) Date of publication of application: 22.11.2023
(73) Proprietor: Ricoh Company, Ltd., Tokyo 143-8555 (JP); National University Corporation Tokyo Medical and Dental University, Tokyo 113-8510 (JP)
(72) Inventor: NAGAOKA, Nobuyori, Tokyo 143-8555 (JP); WATANABE, Taishi, Tokyo 143-8555 (JP); KAWABATA, Shigenori, Tokyo 113-8510 (JP)
(74) Representative: Marks & Clerk LLP
(86) International application number: PCT/JP2022/000596
(87) International publication number: WO 2022/153982

(56) References cited:
- SAKAKI KYOHEI ET AL: "Evaluation of neural activity by magnetospinography with 3D sensors", CLINICAL NEUROPHYSIOLOGY, ELSEVIER, AMSTERDAM, NL, vol. 131, no. 6, 19 March 2020 (2020-03-19), pages 1252 - 1266, XP086150101, ISSN: 1388-2457, [retrieved on 20200319], DOI: 10.1016/J.CLINPH.2020.02.025
- MIYANO YUKI ET AL: "Visualization of electrical activity in the cervical spinal cord and nerve roots after ulnar nerve stimulation using magnetospinography", CLINICAL NEUROPHYSIOLOGY, vol. 131, no. 10, 1 October 2020 (2020-10-01), AMSTERDAM, NL, pages 2460 - 2468, XP055793789, ISSN: 1388-2457, DOI: 10.1016/j.clinph.2020.07.009
- SENICHI ISHII ET AL: "Conductive neuromagnetic fields in the lumbar spinal canal", CLINICAL NEUROPHYSIOLOGY, ELSEVIER, AMSTERDAM, NL, vol. 123, no. 8, 23 December 2011 (2011-12-23), pages 1656 - 1661, XP028503544, ISSN: 1388-2457, [retrieved on 20120103], DOI: 10.1016/J.CLINPH.2011.12.014

## Description

### Technical Field

The present invention relates to a diagnostic support apparatus, diagnostic support method, and diagnostic support program.

### Background Art

For example, as a method of measuring a biomagnetic field, a method of applying electrical stimulation to a part of a subject to induce nerve activity in a part and measuring a magnetic field generated by nerve activity is known. In addition, there are known methods for non-invasively evaluating neural action currents by reconstructing currents generated in vivo using magnetic field data obtained by measuring the biomagnetic field by a sensor.

For example, the conduction velocity of the neural action current in a cauda equina can be computed non-invasively by measuring the neuromagnetic field in a lower back when the peroneal nerve in the knee is stimulated and reconstructing a neural action current from the obtained magnetic field data. In addition, a correlation between the age and the conduction velocity can be obtained by statistical analysis of the conduction velocity of the neural action current in a plurality of healthy subjects using the above-described method (see, for example, Shuta Ushio et al, "Visualization of the electrical activity of the cauda equina using a magnetospinography system in healthy subjects", Clinical Neurophysiology, Volume 130, Issue 1, January 2019, Pages 1-11).

K. Sakaki et al., "Evaluation of neural activity by magnetospinography with 3D sensors", 2020 discloses a magnetospinography system with sensor positions in three orthogonal directions to record lumbar canal evolved magnetic fields in response to peripheral nerve simulation), and methods for localizing spinal lesions. Y. Miyano et al., "Visualisation of electrical activity in the cervical spinal cord and nerve roots after nerve simulation using magnetospinography", 2020 discloses a method for magnetospinography measurement after ulnar nerve stimulation.

### [Summary of Invention]

### [Technical Problem]

However, no method is disclosed for generating information that can assist in diagnosing diseases such as the lumbar spine from the neural action current computed using the magnetic field data obtained by measuring the magnetic field generated by the subject.

### [Solution to Problem]

The disclosed technique has been developed in view of the above problems and is aimed to provide the diagnostic support apparatus, diagnostic support method, and diagnostic support program that can assist in diagnosing neurological diseases.

In order to solve the above-described technical problem, an embodiment of the diagnostic support apparatus of the present invention comprises a computing unit for computing a decreasing rate in the current intensity of an inward current flowing into a nerve pathway, based on magnetic field data obtained by measuring the magnetic field generated from a subject, and a display controller for displaying a comparison result in which the decreasing rate in the current intensity is compared with a standard value, on a display unit, and further comprises a standard changing unit that is configured to change the predetermined standard value based on information indicating a measurement portion for which the current intensity is computed.

Thus, the diagnostic support apparatus, diagnostic support methods, and diagnostic support programs that can assist in diagnosing neurological diseases can be provided.

### Brief Description of Drawings

[Fig.1]Fig. 1 is a diagram illustrating an example of a biometric measurement system including a diagnostic support apparatus according to a first embodiment of the invention.
[Fig.2]Fig. 2 is a perspective view illustrating an example of a SQUID sensor array provided in a protruding unit of a low temperature container illustrated in Fig. 1.
[Fig.3]Fig. 3 is a diagram illustrating an example of a functional block of a data processor illustrated in Fig. 1.
[Fig.4]Fig. 4 is a diagram showing an example of a model of a neural action current.
[Fig.5]Fig. 5 is a diagram illustrating an example of a time change of a current distribution extracted based on a magnetic field data of a measurement target portion (central nervous system) obtained by the biometric measurement system illustrated in Fig. 1.
[Fig.6]Fig. 6 is a diagram illustrating an example of the time change of the current distribution extracted based on the magnetic field data of the measurement target portion (central nervous system) obtained by the biometric measurement system illustrated in Fig. 1.
[Fig.7]Fig. 7 is an example of a change in the current waveform when the inward current observed in Figs. 5 and 6 is conducted from an upstream to a downstream.
[Fig.8]Fig. 8 is a diagram illustrating another display example of abnormal information illustrating a decreasing rate of the current intensity to be displayed on the decreasing rate error display unit illustrated in Fig. 3.
[Fig.9]Fig. 9 is a diagram illustrating an example of a time change in a current distribution extracted based on magnetic field data obtained at a measurement target portion (peripheral nerve) obtained by the biometric measurement system.
[Fig.10]Fig. 10 is a diagram illustrating an example of a change in a current waveform when an inward current observed in Fig. 9 is conducted from the upstream to the downstream.
[Fig.11]Fig. 11 is a block diagram illustrating an example of a hardware structure of the data processor illustrated in Fig. 3

### Mode for Carrying Out the Invention

Hereinafter, embodiments will be described with reference to the drawings. In each drawing, the same components are indicated by the same reference numerals and overlapping descriptions may be omitted.

### Description of Embodiments

### First Embodiment

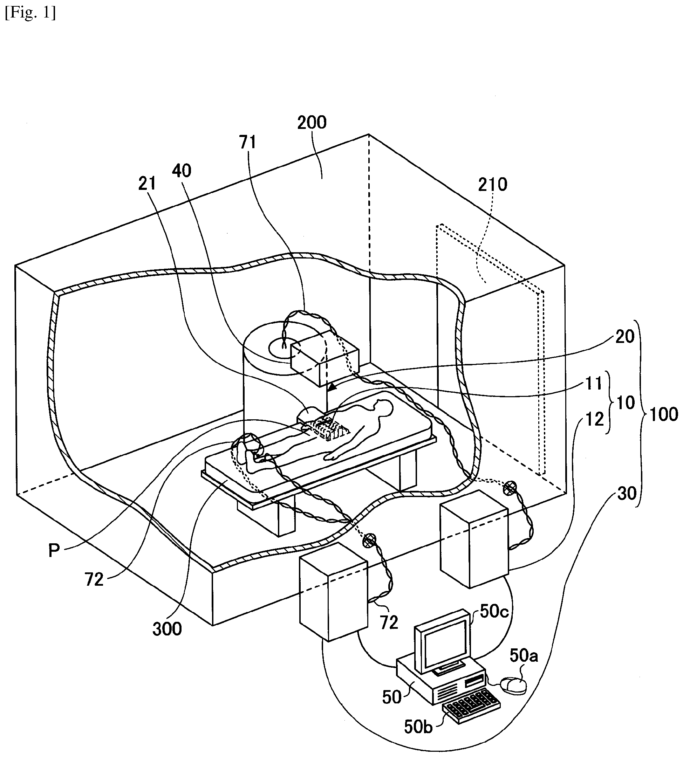

Fig. 1 illustrates an example of a biometric measurement system including a diagnostic support apparatus according to a first embodiment of the present invention. For example, a biometric measurement system 100 illustrated in FIG. 1 includes a spinal cord evoked magnetic field measurement system that measures the magnetic field generated by the nerve, such as the spinal cord, based on electrical stimulation.

The biometric measurement system 100 includes a magnetic field measuring device 10, a low temperature container 20, a nerve stimulator 30, an X-ray device 40, and a data processor 50 as major components. The data processor 50 is an example of a diagnostic support apparatus. The nerve stimulator 30 is a device that electrically stimulates the nerve from the surface of the body (skin) of the subject P. In FIG. 1, the X-ray device 40 is disposed on the top of the subject P, but may be disposed on the side and may be disposed on both the top and the side.

The magnetic field measuring device 10 includes a SQUID sensor array 11 including a plurality of superconducting quantum interference devices (SQUID) and a signal processor 12. The magnetic field measuring device 10 can measure the magnetic field evoked by the nerve of the subject P to be measured upon electrical stimulation by the nerve stimulator 30.

In this embodiment, the magnetic field measuring device 10 is used as a magnetospinograph (MSG). The magnetic field measuring device 10 may be used as a magnetoencephalography (MEG) or a magnetocardiograph (MCG). Hereinafter, the superconducting quantum interference device is also referred to as SQUID.

The data processor 50 has a function of controlling the timing of electrical stimulation to a biological body by the nerve stimulator 30, and has a function of processing information processing of biological information such as a biomagnetic field measured by the magnetic field measuring device 10. The data processor 50 also has a function for controlling the capture of an X-ray image of the subject P by the X-ray device 40. The data processor 50 functions to receive inputs from input/output devices such as a mouse 50a and a keyboard 50b.

The data processor 50 has a function of superimposing the direction of the current generated in response to the magnetic field measured by the magnetic field measuring device 10 on an X-ray image and displaying it on the display 50c. The data processor 50 has the function of calculating the time change of the current value at a plurality of consecutive positions (virtual electrodes) instructed by an operator operating the mouse 50a with respect to an image displayed on the display 50c. For example, the operator may indicate a plurality of locations along a nerve, such as the spinal cord, which is recognized by an X-ray image displayed on the display 50c.

The data processor 50 also has a function to display the current waveform of each virtual electrode on the display 50c. The data processor 50 has a function of displaying a text or a graphic indicating that the decreasing rate of the current intensity, which is the maximum amplitude of the current waveform in adjacent virtual electrodes, is reduced when the decreasing rate is less than a predetermined standard value of the decreasing rate, or the like at a position adjacent to the current waveform.

A portion of the biometric measurement system 100 is disposed within magnetic shielding room 200 that shields the magnet. The magnetic shielding room 200 can be used to measure a weak magnetic field (e.g., spinal cord evoked magnetic field) generated by the subject P. The magnetic shielding room 200 may be formed by laminating, for example, a plate material made of permalloy or the like as a high magnetic permeability material and a plate material made of an electrically conductive material such as copper or aluminum.

The magnetic shielding room 200 has an internal space of, for example, about 2.5 m depth, 3.0 m width, and 2.5 m height, and is provided with a door 210 for transporting devices and instruments and for allowing for entry and exit of persons. The door 210, as well as other portions of the magnetic shielding room 200, may be formed by laminating a plate material made with permalloy or the like as a high magnetic permeability material and a plate material made of an electrically conductive material such as copper or aluminum.

As used herein, a high magnetic permeability material refers to a material having a specific magnetic permeability greater than 1000. Examples of the high magnetic permeability material are iron, nickel, and cobalt alone, alloys thereof (including amorphous alloys, powders, and nanoparticles), ferrites, and the like, in addition to permalloys.

Hereinafter, the biometric measurement system 100 and its peripheral portions will be described in more detail. A bed 300 is provided within the magnetic shielding room 200. The low temperature container 20 is installed in the magnetic shielding room 200, and a signal cable 71 used for measuring the magnetic field and for controlling the measurement is coupled to the SQUID sensor array 11, which is installed in the protruding unit 21 of the low temperature container 20. The signal cable 71 has a twisted cable structure to reduce magnetic field noise and is drawn out of the magnetic shielding room 200 a through hole through the wall portion of the magnetic shielding room 200 and coupled to a signal processor 12.

For example, spinal cord evoked magnetic field measurements using the biometric measurement system 100 are conducted while the subject P lies in the supine position on the bed 300 at rest. When the measurement at rest is performed, not only is the load on the subject P reduced, but the displacement of the subject P from the SQUID sensor array 11 due to the movement of the subject P can be reduced, and the magnetic field noise from the muscle caused by the muscle contraction and the like can be reduced.

The low temperature container 20, also referred to as Dewar, holds the liquid helium necessary to operate the SQUID sensor array 11, which detects a magnetic field generated from the subject P, at an extremely low temperature. The protruding unit 21 of the low temperature container 20 has a shape suitable for measuring spinal cord evoked magnetic fields. For example, the spinal cord evoked magnetic field is measured while the lower back of the subject P is made in contact with the protruding unit 21 and the lower back faces a head of the SQUID sensor array 11.

When measuring the spinal cord evoked magnetic fields, it is necessary to deliberately evoke the neural activity of the subject P by electrical stimulation. Electrical stimulation is applied using the nerve stimulator 30. For example, in this embodiment, electrical stimulation is applied alternately to the left tibial nerve and the right tibial nerve at the ankles, and the current conducted from an upstream to a downstream of the spinal cord is computed based on each electrical stimulation. Here, the term "upstream" refers to a position relatively close to the stimulation position to which the electrical stimulation is applied, and the term "downstream" refers to a position relatively distant from the stimulation position. Therefore, the two electrode pairs coupled to the nerve stimulator 30 via the two signal cables 72 of the twisted cable structure are applied to the skin inside the both ankles.

The tibial nerves of both of the legs of the subject P are alternately excited by electrical stimulation applied to the subject P alternately from an electrode pair, and nerve activity caused by the excitation is propagated to the central nervous system. The SQUID sensor array 11 opposite to the lumbar region of the subject P detects magnetic fields arising from the spinal cord and spinal nerves of the lumbar region.

In addition, the two electrode pairs may be applied to nerves other than the tibial nerves of both of the legs at an electrically stimulable position. Measurement positions of the biomagnetic fields based on electrical stimulation from both of the legs may be the thoracic or cervical region. In addition, when measuring the biomagnetic field in the cervical region, the two pairs of electrodes may be attached to positions capable of stimulating to nerves of both hands location.

By alternately applying electrical stimulation from the two electrode pairs and measuring the biomagnetic field alternately, differences in the current waveforms conducted through the left and right nerves to the spinal cord can be detected. Then, based on the difference in the current waveform, information for identifying a damaged area can be provided to an evaluator such as a physician.

For example, the data processor 50 is a computer, such as a PC (Personal Computer), connected via signal cables to the signal processor 12, the nerve stimulator 30, and the X-ray device 40. The data processor 50 controls the operation of the magnetic field measuring device 10, the nerve stimulator 30, and the X-ray device 40.

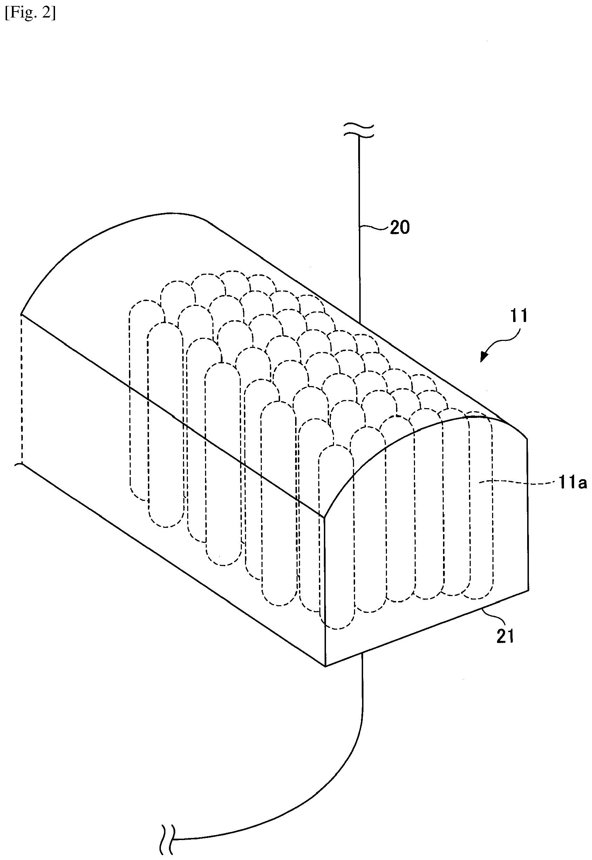

Fig. 2 is a perspective view illustrating an example of the SQUID sensor array 11 provided in protruding unit 21 of the low temperature container 20 illustrated in Fig. 1. For example, the SQUID sensor array 11 has a plurality of SQUID sensors 11a having a vertically extending bar shape and staggered in a top view. The upper end of each SQUID sensor 11a is positioned so as to face a measurement target portion of the subject P lying on the bed 300. In this embodiment, the plurality of SQUID sensors 11a are disposed within the protruding unit 21 such that the upper end is slightly curved in accordance with the shape of the curved shape of the lower back of the subject P during the magnetic field measurement.

Each SQUID sensor 11a measures the magnetic field generated by the subject P based on an instruction from the signal processor 12 and outputs the measured magnetic field as a voltage signal (the magnetic field signal indicating the magnetic field) to the signal processor 12. For example, each SQUID sensor 11a is a three-axis sensor having an X-axis, a Y-axis, and a Z-axis, and the magnetic field signal can be measured as a three-dimensional vector quantity. Each SQUID sensor 11a may be a two-axis sensor having the X-axis and the Y-axis capable of measuring a magnetic field signal as a two-dimensional vector quantity, or may be a single-axis sensor having only the Z-axis.

The signal processor 12 illustrated in Fig. 1 estimates the neural action current at a specified point on the basis of the relationship between the position of the nerve of the subject P on the X-ray image and the position of each SQUID sensor 11a and the magnetic field data obtained by measuring the magnetic field generated from the subject P by the plurality of SQUID sensors 11a. For example, estimation algorithms such as a spatial filtering method are used to estimate the neural action current. This provides a current waveform indicative of the time change of the neural action current at any position facing the SQUID sensor array 11. In order to remove artifacts from the magnetic field data obtained by measuring the magnetic field, the Dual Signal Subspace Projection (DSSP) method, which is an artifact reduction method, or the like may be applied.

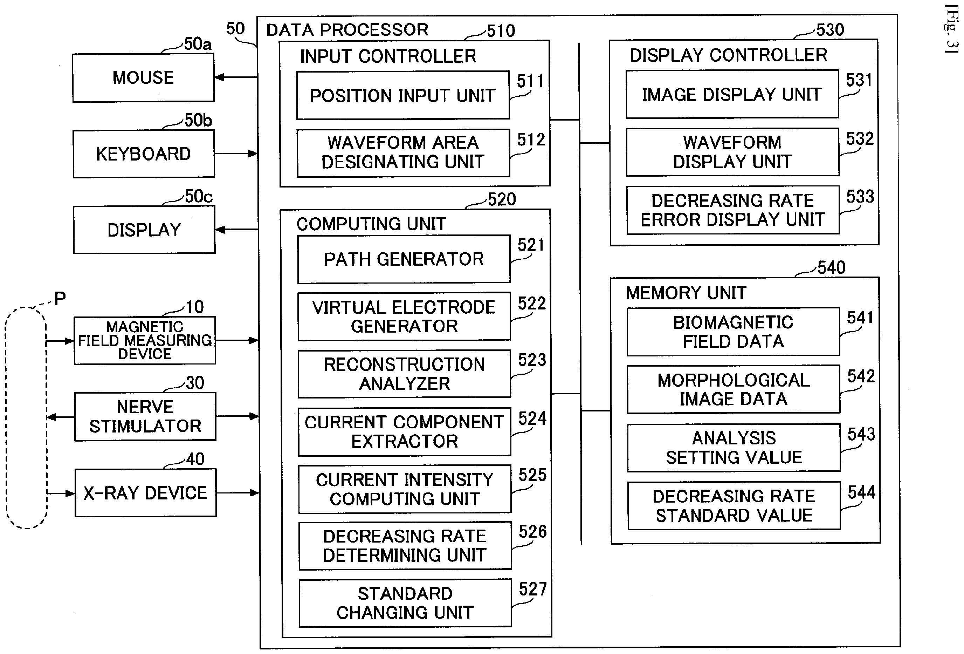

Fig. 3 is a diagram illustrating an example of a functional block of the data processor 50 illustrated in Fig. 1. The data processor 50 includes an input controller 510, a computing unit 520, a display controller 530, and a memory unit 540. For example, the input controller 510, the computing unit 520, and the display controller 530 are implemented by a data processing program executed by a processor such as a CPU (Central Processing Unit) installed in the data processor 50.

The calculation of the decreasing rate of the inward current by the input controller 510, the computing unit 520, and the display controller 530, which will be described later, and the display of a comparison result in which the decreasing rate of the current intensity is compared with the predetermined standard value are performed by executing the diagnostic assistance program from among the data processing programs. Then, when a processor such as the CPU executes the diagnostic support program, the diagnostic support method is executed.

The input controller 510, the computing unit 520, and the display controller 530 may be implemented by hardware, such as an FPGA, or may be implemented by a combination of software and hardware. The computing unit 520 is an example of the computing unit which computes the decreasing rate of the current intensity of the inward current flowing into the nerve pathway based on the magnetic field data obtained by measuring the magnetic field generated by the subject P.

For example, the memory unit 540 may be implemented by at least one of semiconductor memory devices such as a dynamic random access memory (DRAM), static random access memory (SRAM), read only memory (ROM), and flash memory. The memory unit 540 may be implemented by a semiconductor memory device and a hard disk drive (HDD) or a solid state drive (SSD).

The input controller 510 includes a position input unit 511 and a waveform area designating unit 512. The computing unit 520 includes a path generator 521, a virtual electrode generator 522, a reconstruction analyzer 523, a current component extractor 524, a current intensity computing unit 525, a decreasing rate determining unit 526, and a standard changing unit 527. The display controller 530 includes an image display unit 531, a waveform display unit 532, and a decreasing rate error display unit 533. The display controller 530 may be disposed outside the data processor 50. A memory area for storing the biomagnetic field data 541, the morphological image data 542, the analysis setting value 543, and the decreasing rate standard value 544. is allocated to the memory unit 540.

The input controller 510 receives an operation such as a mouse 50a and a keyboard 50b, by an operator of the magnetic field measuring device 10. The position input unit 511 receives the position of a control point for expressing a nerve path, such as the spinal cord, on a morphological image, such as an X-ray image displayed on the display 50c. The position information received by the position input unit 511 is stored in the memory unit 540 as the analysis setting value 543. The waveform area designating unit 512 receives a time range in which the current waveform computed by the virtual electrode is displayed on the display 50c. A virtual electrode set on a nerve path, such as the spinal cord, on a morphological image is an example of a second virtual electrode that is positioned at predetermined intervals along the nerve route.

The path generator 521 computes the path of the active nerve based on position information of the plurality of control points input from the position input unit 511. The set nerve path is called a nerve route. Here, the computed path is represented by a plurality of coordinate information or an expression representing a curve, and is stored in the memory unit 540 as an analysis setting value 543.

The virtual electrode generator 522 generates a plurality of virtual electrodes A at equal intervals on the nerve route computed by the path generator 521. Further, a virtual electrode B is generated on the virtual electrode A in the nerve running at a point at a predetermined distance in a direction normal to the nerve route. The distance and the number of the virtual electrodes generated in the path in the normal direction are specified by an operator through an input device such as a mouse 50a or a keyboard 50b, and stored in the memory unit 540 as an analysis setting value 543 by the input controller 510.

The reconstruction analyzer 523 reconstructs the current components for each voxel arranged like a matrix at predetermined intervals using the magnetic field data of the subject P obtained by the measurement of the biomagnetic field by the magnetic field measuring device 10. The voxel is an example of a first virtual electrode installed in a predetermined region that includes a magnetic field data acquisition site.

The current component extractor 524 extracts the electromagnetic waveform of each virtual electrode using the current component in the voxel computed by the reconstruction analyzer 523 based on the positional relationship between each virtual electrode and the voxel. For example, the current component extractor 524 extracts the current component (the direction from upstream to downstream is positive and the direction from downstream to upstream is negative) at the virtual electrode A along the nerve route in the time range received by the waveform area designating unit 512 as the current in the axon that conducts the nerve axon in the nerve route.

The current component extractor 524 extracts the current component in the normal direction relative to the nerve route on the virtual electrode B as the inward current within the time range received by the waveform area designating unit 512. Here, the direction toward the nerve route is the positive direction. Then, a current waveform of the inward current is generated by a time change in the extracted inward current. Among volume currents flowing outside a nerve axon, the inward current, which is the current component entering a depolarizing region, is important in assessing nerve function. The current waveform is generated, for example, by the waveform display unit 532 arranging inward current values in chronological order to obtain image data. The current component extractor 524 and the waveform display unit 532 are examples of a waveform generator that generate waveforms of inward currents in the virtual electrode.

The current intensity computing unit 525 computes the maximum value of the amplitude of the current waveform of the inward current for each virtual electrode computed by the current component extractor 524 as the current intensity. For example, the current intensity computing unit 525 computes the amplitude of the positive value of the current waveform as the current intensity.

The decreasing rate determining unit 526 computes a percentage reduction in the current intensity of the inward current for each pair of adjacent virtual electrodes. For example, the decreasing rate determining unit 526 computes the decreasing rate (%) of the current intensity by using Eq. (1). In Eq. (1), the sign "*" denotes the operator of product. Decrease in current intensity (%) = ((Downstream current intensity CS2)/(Upstream current intensity CS1))*100...(1)

The decreasing rate determining unit 526 compares the computed decreasing rate of the current intensity with a predetermined standard value (%). For example, the decreasing rate determining unit 526 determines whether the computed decreasing rate of the current intensity is less than a predetermined standard value. When the computed decreasing rate of the current intensity is less than a predetermined standard value (%) of the decreasing rate, the decreasing rate determining unit 526 outputs the computed decreasing rate of the computed current intensity to the decreasing rate error display unit 533 together with the position information of the virtual electrode located at the downstream where the current intensity is computed, for example, as a comparison result. In addition, instead of outputting the comparison result, a computed decreasing rate of the current intensity and a predetermined standard value (a standard value (%) of a predetermined decreasing rate) may be output and displayed on the display 50c.

The standard changing unit 527 modifies the standard value of the decreasing rate based on information representing a measurement portion for computing the current intensity of the inward current input through the input device such as the mouse 50a or the keyboard 50b. For example, the standard value of the decreasing rate for each measurement portion is stored in the memory unit 540 as the decreasing rate standard value 544. Changing the standard value of the decreasing rate enables the standard value to be appropriately set depending on the measurement portion and the spinal disease diagnosis to be appropriately conducted. The standard changing unit 527 may store the standard value of the decreasing rate input from the keyboard 50b or the like as the decreasing rate standard value 544 in the memory unit 540.

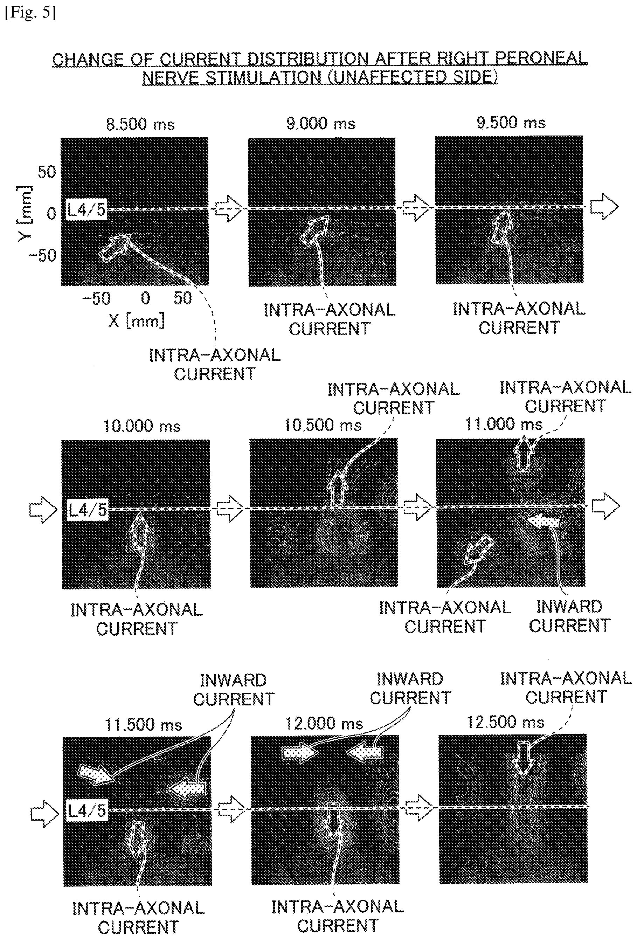

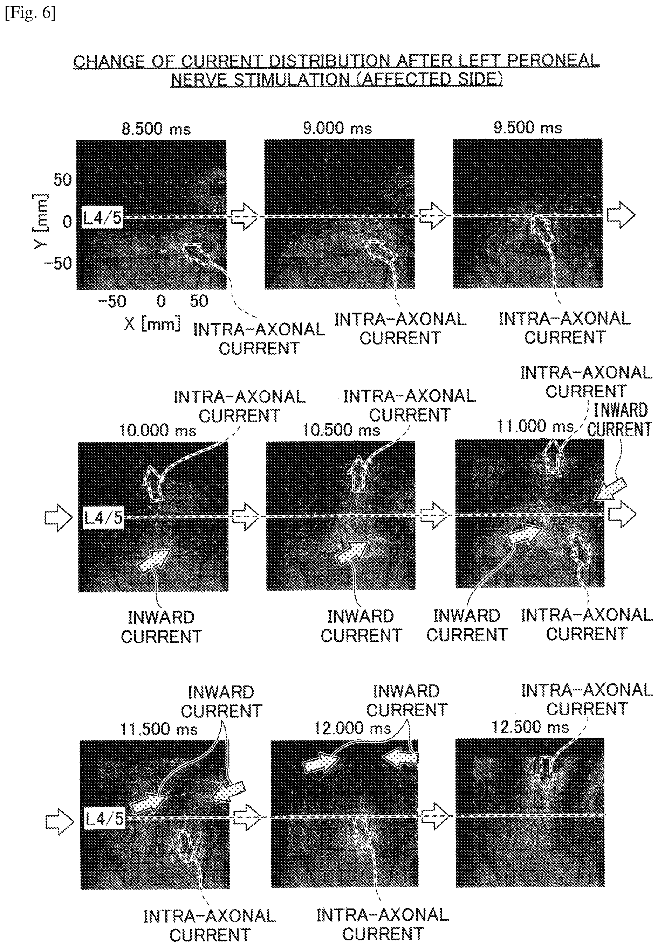

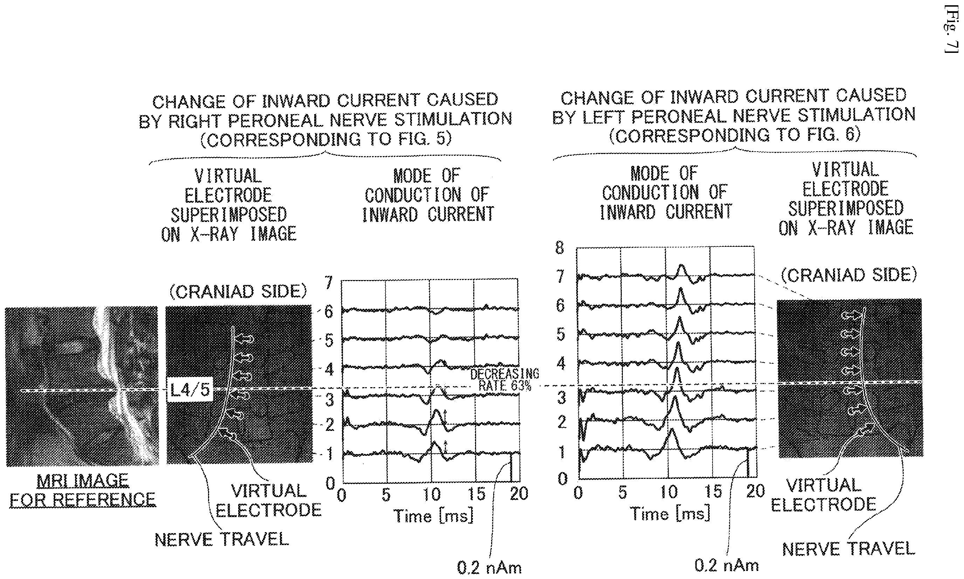

As illustrated in Figs. 5 and 6, the image display unit 531 superimposes a small white arrow representing the direction and intensity of the current in each voxel reconstructed by the reconstruction analyzer 523 on a morphological image (X-ray image) and displays it on the display 50c. As illustrated in Fig. 7, the image display unit 531 superimposes the nerve path computed by the path generator 521 and the virtual electrode generated by the virtual electrode generator 522 on the X-ray image and displays these on the display 50c.

The waveform display unit 532 displays the current waveform of each virtual electrode computed by the current component extractor 524 in accordance with the virtual electrode superimposed on the X-ray image on the display 50c.

Upon receiving the decreasing rate of the current intensity and the position information of the virtual electrode from the decreasing rate determining unit 526, the decreasing rate error display unit 533 displays abnormal information indicating that the decreasing rate of the current intensity is erroneous on the display 50c. For example, the error information is displayed on the current waveform or beside the current waveform of the virtual electrode indicated by the position information received from the decreasing rate determining unit 526 as a value of the decreasing rate, a text such as an "erroneous decreasing rate" or the like, or a flickering graphic or the like.

The memory area of the biomagnetic field data 541 stores the magnetic field data obtained by measuring the magnetic field generated from the subject P by the magnetic field measuring device 10. The memory area of the morphological image data 542 stores the X-ray image data of the magnetic field of the subject P captured by the X-ray device 40.

In the memory area for the analysis setting value 543, various parameters required for the measurement of the biomagnetic field by the magnetic field measuring device 10 and various set values such as filters (a high pass filter, low pass filter, and so on) used for the magnetic field data obtained by the measurement of the biomagnetic field are stored in advance. In the memory area of the analysis setting value 543, the position information representing the position of the voxel which is the calculation point of the current in the image displayed on the display 50c and the position of the virtual electrode which acquires the current waveform is stored in advance.

The standard value of the current intensity to be referred by the decreasing rate determining unit 526 is stored in advance in the memory area for the decreasing rate standard value 544. Although it is not limited, for example, the standard value of the decreasing rate in the lumbar is 70%. Provided that the distance between the virtual electrodes is n (mm), the value of n-power (percentage) of 0.97 is defined as the standard value of the decreasing rate, and the decreasing rate of the current intensity may be set according to the distance between the virtual electrodes. By setting the standard value of the decreasing rate of the current intensity at the distance between the virtual electrodes of a few millimeters, comparison of the current with the standard value can be made at a spatial resolution of a few millimeters.

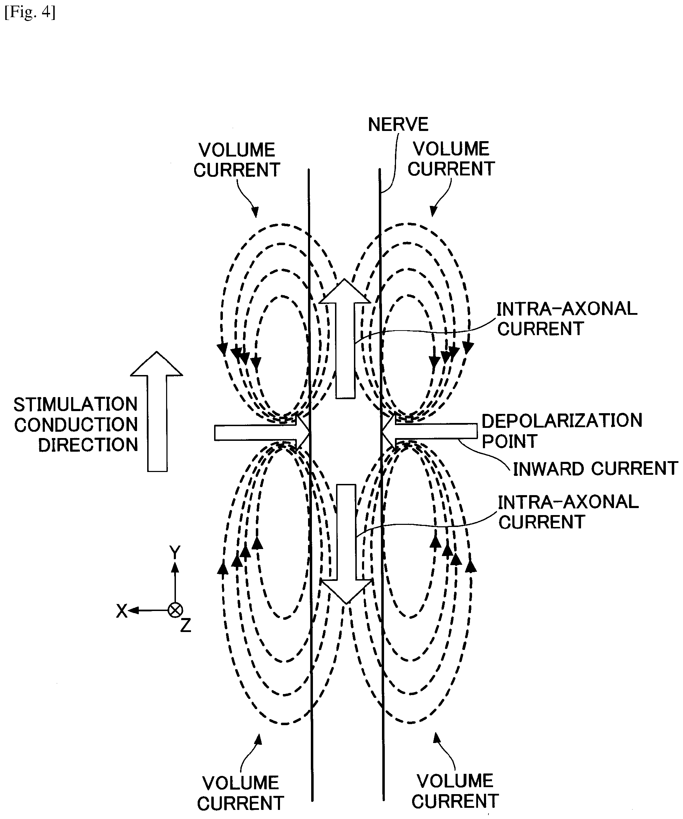

Fig. 4 is a diagram illustrating an example of a model of a neural action current. Fig. 4 illustrates how the current is generated, by the nerve activity, to linearly run in the up and down directions in the figure. The lower side of Fig. 4 is the distal side and the upper side of Fig. 4 is the proximal side. For example, by applying electrical stimulation to the peripheral nerve, the stimulation conducts through the nerve axon from the lower side to the upper side as an electrical current.

At this time, an intra-axonal current flowing toward the upper (forward) side of Fig. 4 and an intra-axonal current flowing toward the lower (reverse) side of Fig. 4 and a volume current, which is the current component flowing out of the nerve axon and returning to the depolarization point, are generated. The intra-axonal current flowing toward the upper side of FIG. 4 is referred to as a leading component, and the intra-axonal current flowing toward the lower side of FIG. 4 is referred to as a trailing component.

For a detailed assessment of nerve function, it is preferable to extract and visually display on display 50c an intra-axonal current flowing along the nerve axon, i.e., a current component in the direction along the nerve route, and the inward current flowing into the depolarization point, i.e., the current component in the direction toward the nerve route from the normal direction of the nerve route.

Fig. 5 and Fig. 6 are diagrams illustrating an example of the time change of the current distribution extracted based on the magnetic field data of the measurement target portion (central nervous system) acquired by the biometric measurement system 100 of Fig. 1. The images illustrated in Figs. 5 and 6 are images in which the current components of each voxel reconstructed by the reconstruction analyzer 523 are superimposed on the X-ray image by the image display unit 531 and are displayed on the screen of the display 50c at one time. Any one from among the image of FIG. 5 and the image of FIG. 6 may be displayed on the screen of the display 50c, and the image for a specified time may be enlarged.

The image in FIG. 5 illustrates, for example, the time change of the current distribution computed from the magnetic field data obtained by measuring the biomagnetic field generated when the electrical stimulation is applied to the right tibial nerve at the ankle of the subject P, which may have a disc herniation. The image in FIG. 6 illustrates the time change of the current distribution computed from the magnetic field data obtained when the electrical stimulation is applied to the left tibial nerve at the ankle of the same subject P. In the images illustrated in Figs. 5 and 6, the current components are superimposed on the X-ray image of the subject P lying on the bed 300 in the supine position when captured from above. For this reason, the left side of the image at each time corresponds to the right side of the subject P, and the right side of the image at each time corresponds to the left side of the subject P.

A time, such as "8.500 ms" illustrated on the upper side of each image in FIGS. 5 and 6, indicates the elapsed time after applying the electrical stimulation. The thick dotted line arranged in the lateral direction of each image represents the junction (L4/5) of the fourth lumbar spine (L4) and the fifth lumbar spine (L5). The arrows arranged between the images for each time are added to make the passage of the time easy to understand, and are not required to be displayed on the screen of the display 50c.

In each image, a plurality of small white arrows indicates the direction of current per voxel extracted by reconstruction, and the length of the arrow indicates the current intensity. At the arrow, the end opposite to an arrow tail is the position of the voxel, which is an extraction unit of the current component. The contour-like curve is a current intensity distribution line produced by connecting positions where the current intensities are the same.

In the image at each time, dotted arrows indicate intra-axonal current and shaded arrows indicate the inward current. The dotted arrows and shaded arrows are provided for illustration purposes and are not included in the image displayed on the display 50c.

In order to obtain the magnetic field data used to calculate the current components illustrated in Figs. 5 and 6, the subject P is laid in the supine position on the bed 300 of Fig. 1 so that the lower back of the subject P faces the SQUID sensor array 11. Then, a plain X-ray image of the lower back of the subject P is captured, and the positional relationship between each SQUID sensor 11a and the lumbar of the subject P is acquired by the data processor 50.

Next, an electrode of the nerve stimulator 30 is applied to the portions of the ankles of both legs, and the electrical stimulation (a 5 Hz square wave pulse with a duration of 0.3 ms) is applied alternately to the left and right tibial nerves. The neuromagnetic field generated in the lower back in response to the electrical stimulation is then measured by the magnetic field measuring device 10.

FIG. 5 illustrates the time change of the current distribution when the right tibial nerve is electrically stimulated. It is observed that the leading and trailing components of the intra-axonal current intruded through the L5 intervertebral foramen on the right side of the subject P (left in FIG. 5) are conducted downstream, as indicated by the dotted arrows. As indicated by the shaded arrows, inward currents are observed to be directed toward the nerve axon.

Referring to Fig. 6 illustrating the time change of the current distribution when the left tibial nerve is electrically stimulated, as indicated by the dotted arrows, it is observed that the leading and trailing components of the intra-axonal current intruding through the L5 intervertebral foramen of the left side (the right side in Fig. 6) of the subject P are conducted to the downstream. As indicated by the shaded arrows, the inward currents are observed to be generated toward the nerve axon.

Fig. 7 is a diagram illustrating an example of the change in the current waveform when the inward current observed in Figs. 5 and 6 is conducted from the upstream to the downstream. The current waveform illustrated in Fig. 7 is computed on the virtual electrode by the current component extractor 524. The waveform of the inward current corresponding to Fig. 5 illustrates a decrease in the current intensity at the position of the L4/5 intervertebral disk.

The arrows indicated in the waveform of the inward current corresponding to Fig. 5 are added for clarity of explanation. The arrows indicate the positive amount of the amplitude amount (current intensity) of the current waveform used for computing the decreasing rate.

In the two X-ray images illustrated in Fig. 7, the virtual electrodes are displayed so as to be superimposed on the proximal side of the body with respect to the nerve route added for reference. In the X-ray images, the arrows pointing toward the nerve from the virtual electrode indicate the inward current. In this manner, the virtual electrodes for extracting the inward current are spaced apart by a predetermined distance from the nerve route. The virtual electrode for extracting the inward current is set to the side opposite to the side on which the electrical stimulation is applied. For example, in the X-ray image corresponding to Fig. 5, the tibial nerve of the right ankle (left side in Fig. 7) is electrically stimulated, so a virtual electrode is set on the left side of the subject P (right side in Fig. 7) with respect the nerve route.

The decreasing rate determining unit 526 determines that the decreasing rate of the current intensity at the position of the L4/5 intervertebral disc is lower than the standard value of the decreasing rate and outputs the decreasing rate of the current intensity to the decreasing rate error display unit 533 together with the position information of the virtual electrode corresponding to the location of the L4/5 intervertebral disc. The decreasing rate of the current intensity is computed by the ratio of the current intensity at the virtual electrode of interest to the current intensity at one upstream virtual electrode (at the position of the L5/S1 intervertebral disc, in this example), as represented by Eq. (1).

The decreasing rate error display unit 533 displays information representing the decreasing rate of the current intensity received from the decreasing rate determining unit 526 as abnormal information on the display 50c adjacent to the corresponding current waveform. In the example illustrated in Fig. 7, 63%, which is the reduction rate computed by the decreasing rate determining unit 526, is displayed on the display 50c as the text "decreasing rate 63%".

Text indicating the decreasing rate may be more prominent or may be flashing than the color of the waveform.

Further, the display of the decreasing rate is not limited to text, and may be a graphic, a pop-up, or the like. The location where the decreasing rate is displayed is not limited to a position adjacent to the waveform, if this location can correspond to the waveform. For example, the decreasing rate may be displayed at the location that is beside the virtual electrode that is superimposed on the X-ray image. Further, the location where the decreasing rate is displayed may be on the upper or lower portion of the image, if the location can correspond to the waveform. In this case, the color of the virtual electrode corresponding to the decreasing rate to be displayed may be different from the color of another virtual electrode, and the graphic of the corresponding virtual electrode may blink.

On the other hand, the waveform of the inward current corresponding to Fig. 6 does not show a decrease in the current intensity at the position of the L4/5 intervertebral disc. Therefore, the decreasing rate determining unit 526 does not output the decreasing rate of the current intensity to the decreasing rate error display unit 533. Since the waveform illustrated in No. 7 is the current waveform not subject to diagnosis, the decreasing rate determining unit 526 does not determine the decrease in the current intensity.

In the image corresponding to Figs. 5 and 6, a plurality of decreasing rates respectively corresponding to all the virtual electrodes that generate the current waveforms may be displayed adjacent to the corresponding waveform or corresponding virtual electrode. In this case, the color displayed on the screen may be changed, or the font size or font type may be changed, depending on whether the decreasing rate in the current intensity is less than the standard value. Thus, the evaluator can recognize the change in the decreasing rate of the current intensity.

The Magnetic Resonance (MR) image illustrated in the left side of Fig. 7 is for reference only and is not necessarily displayed on the display 50c. The display controller 530 displays a superimposed X-ray image and an inward current waveform corresponding to the virtual electrodes of Figs. 5 and 6 in the display 50c. The evaluator such as a physician observing the image illustrated in FIG. 7 is able to recognize that the electrical stimulation of the right tibial nerve decreases the current intensity at the position of the L4/5 intervertebral disc.

The evaluator then examines the MR image, etc. separately captured with reference to the image illustrated in Fig. 7 to diagnose the presence or absence of herniation of L4/5 intervertebral disc, etc. For example, the evaluator who viewed the image illustrated in Fig. 7 can recognize the image illustrated in Fig. 5 as an image on the unaffected side having a disorder, and can recognize the image illustrated in Fig. 6 as the image on the affected side without having a disorder. This allows the evaluator to diagnose, for example, the L4/5 intervertebral disc protruding predominantly to the right side on MR images to be the disc herniation. That is, the image illustrated in FIG. 7 generated by data processor 50 can assist in diagnosing spinal disease.

In evaluating peripheral nerves, the standard value of the current intensity referred by the decreasing rate determining unit 526 is stored in advance in the memory area of the decreasing rate standard value 544. For example, the standard value of the decreasing rate in the palmar area is 70%, although it is not particularly limited. When the distance between the virtual electrodes is n (mm), the value of n-power (percentage) of 0.97 is defined as the standard value of the decreasing rate, and the decreasing rate of the current intensity may be set in response to the distance between the virtual electrodes. By setting the standard value of the decreasing rate of the current intensity at the distance between the virtual electrodes of a few millimeters, the comparison with the standard value can be made at a spatial resolution of a few millimeters.

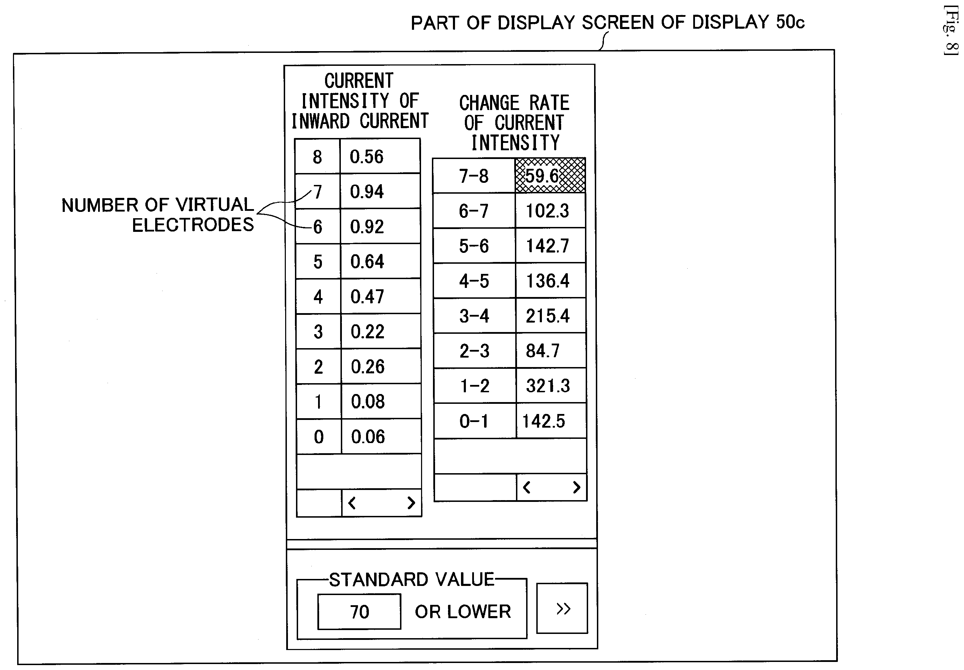

Fig. 8 is a diagram illustrating another display example of abnormal information representing a decreasing rate of the current intensity displayed in the decreasing rate error display unit 533 illustrated in Fig. 3. For example, the decreasing rate determining unit 526 in Fig. 3 computes a percentage reduction in the current intensity of the inward current for each pair of adjacent virtual electrodes. The decreasing rate error display unit 533 displays the value of the current intensity of each virtual electrode, which is computed by the current intensity computing unit 525 illustrated in Fig. 3 and is represented by 0 to 8, on the display 50c. The decreasing rate error display unit 533 displays the rate of change (the decreasing rate) of the current intensity determined by the decreasing rate determining unit 526 in association with the value of the current intensity of each virtual electrode on the display 50c. When the change rate of the current intensity is less than 100, it indicates that the current intensity is decreased.

At this time, the decreasing rate error display unit 533 displays the decreasing rate lower than the standard value previously stored in the memory area of the decreasing rate standard value 544 on the display 50c so as to distinguish it from the decreasing rate higher than or equal to the standard value. For example, the decreasing rate can be distinguished by highlighting, such as by separating colors or changing the character type. Thus, the evaluator can recognize the transition of the decreasing rate of the current intensity while checking the actual current intensity of each virtual electrode and the change rate of the current intensity between each virtual electrode pair.

As illustrated in Fig. 8, the decreasing rate error display unit 533 may display the standard value on the display 50c together with the current intensity and the change rate. The standard value may also be displayed on the display 50c in a modifiable manner on a display screen by a user interface. In this case, when the standard value is changed, the decreasing rate error display unit 533 highlights a rate lower than the standard value after the change on the display 50c in real time.

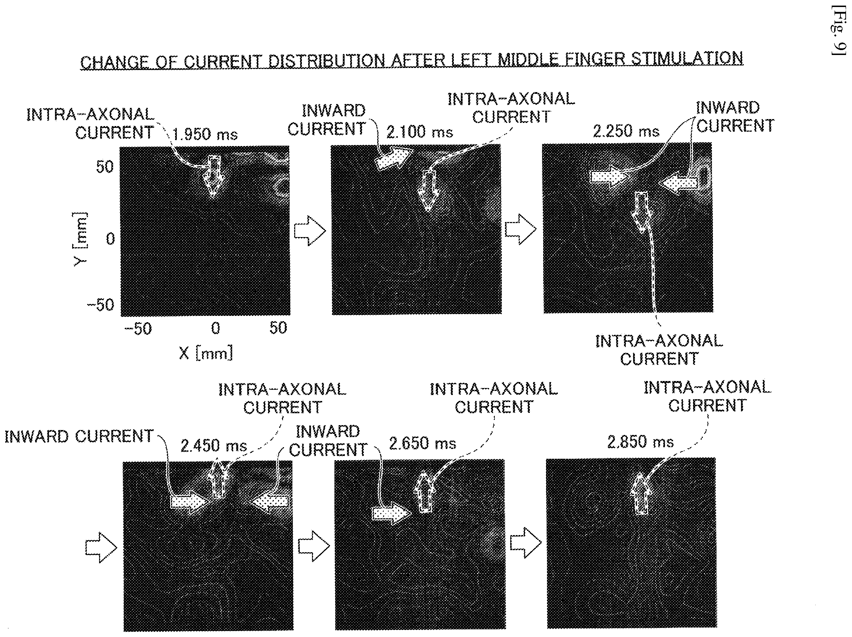

Fig. 9 is a diagram illustrating an example of a time change of a current distribution extracted based on magnetic field data of a target portion (peripheral nerve) acquired by the biometric measurement system 100 of Fig. 1.

For elements similar to FIGS. 5 and 6, the detailed description is omitted. FIG. 8 is the image illustrating the time change of the current distribution computed from the magnetic field data obtained by measuring the biomagnetic field generated when the electrical stimulation is applied to the left middle finger of the subject P, which may have carpal tunnel syndrome.

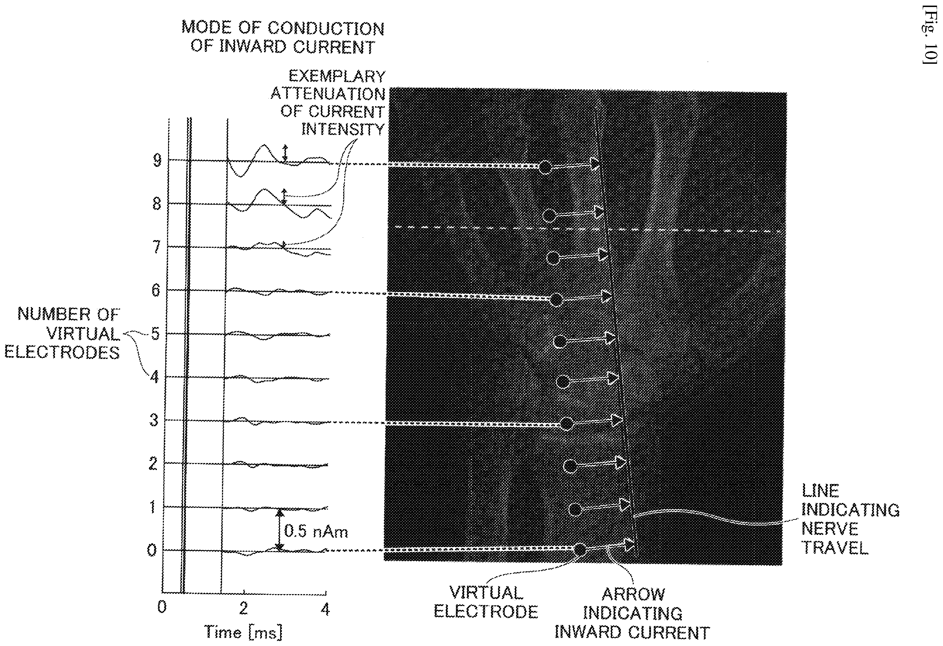

Fig. 10 is a diagram illustrating an example of a change in the current waveform when the inward current observed in Fig. 9 is conducted from the upstream to the downstream. The current waveform illustrate in Fig. 10 illustrates the current waveform at the virtual electrode disposed from the third metacarpal bone along the median nerve and extracted by the current component extractor 524 illustrated in Fig. 3. In FIG. 10, it is recognizable on the screen of the display 50c that the amplitude is attenuated to 36.9% between the waveform 8 representing the inward current and the waveform 7 (near the center of the third metacarpal). In Fig. 10, a virtual electrode superimposed on the X-ray image, the arrow indicating the inward current, and a line representing the nerve route are added to make the explanation easier to understand and are similar to the superimposed image in Fig. 7.

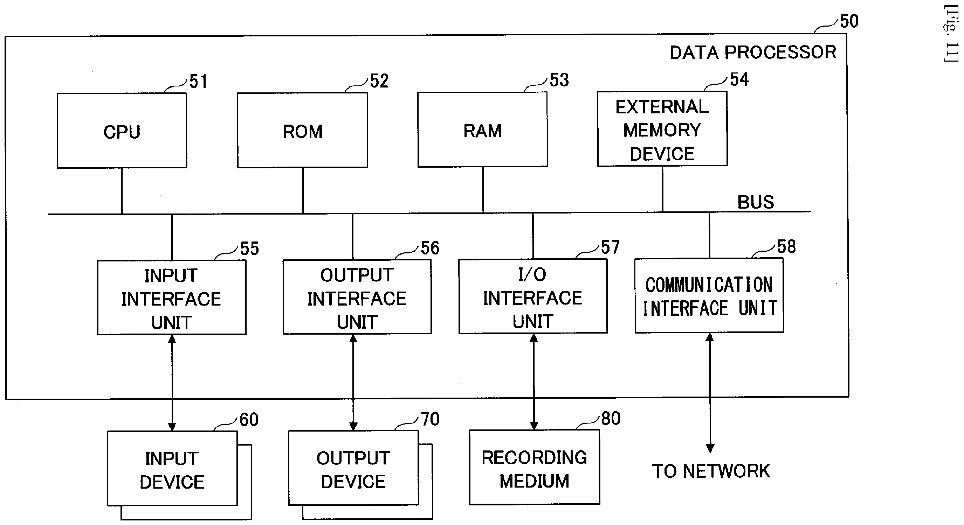

Fig. 11 is a block diagram illustrating an example of a hardware configuration of the data processor 50 of Fig. 3.

The data processor 50 includes a CPU 51, a ROM 52, a RAM 53, and an external memory device 54. The data processor 50 includes an input interface unit 55, an output interface unit 56, an input/output interface unit 57, and a communication interface unit 58. For example, the CPU 51, the ROM 52, the RAM 53, the external memory device 54, the input interface unit 55, the output interface unit 56, the input/output interface unit 57, and the communication interface unit 58 are coupled to each other through the bus BUS.

The CPU 51 executes various programs such as an operating system (OS) and an application and controls the overall operation of the data processor 50. The CPU 51 executes the diagnostic support method by executing the above-described diagnostic support program. The ROM 52 holds various programs and parameters including a diagnostic support program executed by the CPU 51. The RAM 53 stores various programs executed by the CPU 51 and data used in the programs. The external memory device 54 is an HDD or an SSD or the like and stores various programs deployed in the RAM 53.

The input interface unit 55 is coupled to the input device 60 that receives an input from an operator or the like operating the data processor 50. For example, the input device 60 may be a mouse 50a, keyboard 50b, or tablet illustrated in FIG. 3, or the like. An output device 70 for outputting various images, text or graphics generated by the data processor 50 is coupled to the output interface unit 56. For example, the output device 70 may be a display 50c (FIG. 3) or a printer for displaying a display screen or the like generated by various programs executed by the CPU 51.

A recording medium 80, such as a USB (Universal Serial Bus) memory, is coupled to the input/output interface unit 57. For example, various programs, such as diagnostic support programs, may be stored in the recording medium 80. In this case, various programs are transmitted from the recording medium 80 to the RAM 53 through the input/output interface unit 57. The recording medium 80 may be a CD-ROM, a Digital Versatile Disc (DVD) ("Digital Versatile Disc" is a registered trademark), or the like. In this case, the input/output interface unit 57 includes an interface corresponding to the recording medium 80 to be coupled.

The communication interface unit 58 connects the data processor 50 to the network or the like.

Thus, in this embodiment, the decreasing rate of the current intensity of the inward current can be computed based, for example, on the magnetic field data obtained from measurements of the magnetic fields generated from the lower back or the cervical region. Therefore, the diagnosis of spinal disease, such as the disc herniation, can be assisted.

By displaying on the display 50c the decreasing rate of the current intensity smaller than the standard value corresponding to the inward current waveform, the change in the current waveform and the decreasing rate can be provided to the evaluator as a diagnostic aid. For example, when the decreasing rate is less than the standard value, the information representing the decreasing rate corresponding to the current waveform can be displayed on the display 50c, so that an abnormal site can be more easily recognized than when the information representing all computed decreasing rate is displayed.

In addition, it is possible to change the desirable standard decrease rate according to the measurement portion based on the information indicating the measurement portion where the current intensity of the inward current is computed. Therefore, it is possible to desirably support the diagnosis of spinal disease.

Although the invention has been described in accordance with the embodiments, the invention is not limited to the requirements described in the embodiments. In these respects, the subject matter of the present invention may be varied without prejudice and may be suitably defined according to its application.

The present application is based on Japanese Priority Patent Applications No. 2021-003138 filed on January 12, 2021 and No. 2021-093758 filed on June 3, 2021 with the Japan Patent Office.

### [Reference Signs List]

10 Magnetic field measuring device
11: SQUID sensor array
11a: SQUID sensor
12: Signal processor
20: Low temperature container
21: Protruding part
30: Nerve stimulator
40: X-ray equipment
50: Data processor
50a: Mouse 50b: Keyboard 50c: Display
54: External memory device
55: Input interface unit
56: Output interface unit
57: I/O interface unit
58: Communication interface unit
60: Input device 70: Output device
71, 72: Signal wire
80: Recording medium
100: Biometric measurement system
200: Magnetic shielding room
210: Door 300: Bed
510: Input controller
511: Position input unit
512: Waveform area designating unit
520: Computing unit
521: Path generator
522: Virtual electrode generator
523: Reconstruction analyzer
524: Current component extractor
525: Current intensity computing unit
526: Decreasing rate determining unit
527 Standard changing unit
530: Display controller
531: Image display unit
532: Waveform display unit
533: Decreasing rate error display
540: Memory unit
541: Biomagnetic field data
542: Morphological image data
543: Analysis setting value
544 Decreasing rate standard value
BUS: Bus
CS1, CS2: Current intensity
P: Subject

## Claims

1. A diagnostic support apparatus comprising:
a computing unit (520) that computes a decreasing rate of a current intensity of an inward current flowing into a nerve pathway, based on magnetic field data obtained by measuring a magnetic field generated by a subject; and
a display controller (530) that displays a comparison result, in which the decreasing rate of the current intensity is compared with a predetermined standard value, on a display unit;
and **characterized by** further comprising:
a standard changing unit (527) that is configured to change the predetermined standard value based on information indicating a measurement portion for which the current intensity is computed.

2. The diagnostic support apparatus according to claim 1, the diagnostic support apparatus further comprising:
a decreasing rate determining unit (526) that is configured to determine whether the decreasing rate of the current intensity computed by the computing unit is smaller than the predetermined standard value,
wherein the display controller (530) displays, on the display unit, information indicating a decreasing rate of the current intensity that is determined to be less than the predetermined standard value by the decreasing rate determining unit as the comparison result.

3. A diagnostic support apparatus according to claim 2, the diagnostic support apparatus further comprising:
a reconstruction analyzer (523) that reconstructs current components of a plurality of first virtual electrodes installed in a predetermined region including a portion where the magnetic field data are acquired based on the magnetic field data;
a current component extractor (524) that extracts current components in a plurality of second virtual electrodes arranged at predetermined intervals along the nerve pathway on a morphological image of a subject, based on the current components reconstructed by the reconstruction analyzer; and
a waveform generator (532) that generates a waveform of the inward current in the plurality of second virtual electrodes, based on the current components extracted by the current component extractor,
wherein the display controller (530) associates the current waveform generated by the waveform generator with the comparison result and causes the display unit.

4. The diagnostic support apparatus according to claim 3,
wherein the display controller (530) is configured to output, to the display unit, information representing the decreasing rate of the current intensity that is determined to be less than the predetermined standard value by the decreasing rate determining unit together with the corresponding current waveform.

5. The diagnostic support apparatus according to claim 1,
wherein the decreasing rate of the current intensity is expressed as a ratio of CS2/CS1, where a current intensity on a downstream of nerve activity is represented by CS2 and a current intensity on an upstream of nerve activity is represented by CS1.

6. The diagnostic support apparatus according to any one from among claims 1-5,
wherein the computing unit (520) is configured to compute the decreasing rate of the current intensity, based on the magnetic field data obtained by measuring the magnetic field generated from a lower back or a cervical region of the subject.

7. The diagnostic support apparatus according to any one from among claims 1-5,
wherein the computing unit is configured to compute the decreasing rate of the current intensity based on the magnetic field data obtained by measuring the magnetic field generated in a peripheral nerve of the subject.

8. A diagnostic support method comprising:
computing a decreasing rate of a current intensity of an inward current flowing into a nerve pathway, based on magnetic field data obtained by measuring a magnetic field generated by a subject; and
displaying a comparison result, in which the decreasing rate of the current intensity is compared with a predetermined standard value, on a display unit;
and **characterized by** further comprising:
changing the predetermined standard value based on information indicating a measurement portion for which the current intensity is computed.

9. A diagnostic support program comprising program instructions configured to cause a computer to:
compute a decreasing rate of a current intensity of an inward current flowing into a nerve pathway, based on magnetic field data obtained by measuring a magnetic field generated by a subject; and
display a comparison result, in which the decreasing rate of the current intensity is compared with a predetermined standard value, on a display unit;
and **characterized by** further comprising:
change the predetermined standard value based on information indicating a measurement portion for which the current intensity is computed.

## Patentansprüche

1. Diagnoseunterstützungsgerät, Folgendes umfassend:
eine Recheneinheit (520), die eine abnehmende Rate einer Stromstärke eines in eine Nervenbahn fließenden einwärts gerichteten Stroms berechnet, basierend auf Magnetfelddaten, die durch Messen eines von einem Probanden erzeugten Magnetfelds erlangt werden; und
eine Anzeigesteuerung (530), die ein Vergleichsergebnis anzeigt, bei dem die abnehmende Rate der Stromstärke mit einem vorbestimmten Standardwert verglichen wird, auf einer Anzeigeeinheit;
und **dadurch gekennzeichnet, dass** es ferner Folgendes umfasst:
eine Standardänderungseinheit (527), die konfiguriert ist, um den vorbestimmten Standardwert basierend auf Informationen zu ändern, die einen Messabschnitt angeben, für den die Stromstärke berechnet wird.

2. Diagnoseunterstützungsgerät nach Anspruch 1, wobei das Diagnoseunterstützungsgerät ferner Folgendes umfasst:
eine Bestimmungseinheit für die abnehmende Rate (526), die konfiguriert ist, um zu bestimmen, ob die abnehmende Rate der Stromstärke, die von der Recheneinheit berechnet wird, kleiner als der vorbestimmte Standardwert ist,
wobei die Anzeigesteuerung (530) auf der Anzeigeeinheit Informationen anzeigt, die eine abnehmende Rate der Stromstärke angeben, die von der Bestimmungseinheit für die abnehmende Rate als kleiner als der vorbestimmte Standardwert bestimmt wird, als das Vergleichsergebnis.

3. Diagnoseunterstützungsgerät nach Anspruch 2, wobei das Diagnoseunterstützungsgerät ferner Folgendes umfasst:
einen Rekonstruktionsanalysator (523), der Stromkomponenten einer Vielzahl von ersten virtuellen Elektroden rekonstruiert, die in einem vorbestimmten Bereich installiert sind, der einen Abschnitt einschließt, in dem die Magnetfelddaten auf der Grundlage der Magnetfelddaten erfasst werden;
einen Stromkomponenten-Extraktor (524), der Stromkomponenten aus einer Vielzahl von zweiten virtuellen Elektroden extrahiert, die in vorbestimmten Intervallen entlang der Nervenbahn auf einem morphologischen Bild eines Probanden angeordnet sind, basierend auf den Stromkomponenten, die durch den Rekonstruktionsanalysator rekonstruiert werden; und
einen Wellenformgenerator (532), der eine Wellenform des einwärts gerichteten Stroms in der Vielzahl von zweiten virtuellen Elektroden erzeugt, basierend auf den Stromkomponenten, die durch den Stromkomponenten-Extraktor extrahiert werden,
wobei die Anzeigesteuerung (530) die von dem Wellenformgenerator erzeugte Stromwellenform dem Vergleichsergebnis zuordnet und die Anzeigeeinheit veranlasst.

4. Diagnoseunterstützungsgerät nach Anspruch 3,
wobei die Anzeigesteuerung (530) konfiguriert ist, um an die Anzeigeeinheit Informationen auszugeben, welche die abnehmende Rate der Stromstärke darstellen, die von der Bestimmungseinheit für die abnehmende Rate als kleiner als der vorbestimmte Standardwert bestimmt wird, gemeinsam mit der entsprechenden Stromwellenform.

5. Diagnoseunterstützungsgerät nach Anspruch 1,
wobei die abnehmende Rate der Stromstärke als ein Verhältnis von CS2/CS1 ausgedrückt wird, bei dem eine Stromstärke stromabwärts von einer Nervenaktivität durch CS2 dargestellt wird und eine Stromstärke stromaufwärts von einer Nervenaktivität durch CS1 dargestellt wird.

6. Diagnoseunterstützungsgerät nach einem der Ansprüche 1 bis 5,
wobei die Recheneinheit (520) konfiguriert ist, um die abnehmende Rate der Stromstärke auf der Grundlage der Magnetfelddaten zu berechnen, die durch Messen des Magnetfelds erlangt werden, das von einem unteren Rücken oder einem Halsbereich des Probanden erzeugt wird.

7. Diagnoseunterstützungsgerät nach einem der Ansprüche 1 bis 5,
wobei die Recheneinheit konfiguriert ist, um die abnehmende Rate der Stromstärke basierend auf den Magnetfelddaten zu berechnen, die durch Messen des in einem peripheren Nerv des Probanden erzeugten Magnetfelds erlangt werden.

8. Diagnoseunterstützungsverfahren, Folgendes umfassend:
Berechnen einer abnehmenden Rate einer Stromstärke eines in eine Nervenbahn fließenden einwärts gerichteten Stroms, basierend auf Magnetfelddaten, die durch Messen eines von einem Probanden erzeugten Magnetfelds erlangt werden; und
Anzeigen eines Vergleichsergebnisses, bei dem die abnehmende Rate der Stromstärke mit einem vorbestimmten Standardwert verglichen wird, auf einer Anzeigeeinheit;
und **dadurch gekennzeichnet, dass** es ferner Folgendes umfasst:
Ändern des vorbestimmten Standardwerts, basierend auf Informationen, die einen Messabschnitt angeben, für den die Stromstärke berechnet wird.

9. Diagnoseunterstützungsprogramm, umfassend Programmanweisungen, die konfiguriert sind, um einen Computer zu Folgendem zu veranlassen:
Berechnen einer abnehmenden Rate einer Stromstärke eines in eine Nervenbahn fließenden einwärts gerichteten Stroms, basierend auf Magnetfelddaten, die durch Messen eines von einem Probanden erzeugten Magnetfelds erlangt werden; und
Anzeigen eines Vergleichsergebnisses, bei dem die abnehmende Rate der Stromstärke mit einem vorbestimmten Standardwert verglichen wird, auf einer Anzeigeeinheit;
und **dadurch gekennzeichnet, dass** es ferner Folgendes umfasst:
Ändern des vorbestimmten Standardwerts basierend auf Informationen, die einen Messabschnitt angeben, für den die Stromstärke berechnet wird.

## Revendications

1. Appareil d'aide au diagnostic comprenant :
une unité de calcul (520) qui calcule un taux diminuant d'une intensité de courant d'un courant entrant circulant dans un trajet nerveux, sur la base de données de champ magnétique obtenues en mesurant un champ magnétique généré par un sujet ; et
un contrôleur d'affichage (530) qui affiche un résultat de comparaison dans lequel le taux diminuant de l'intensité de courant est comparé à une valeur standard prédéterminée, sur une unité d'affichage ;
et **caractérisé en ce qu'**il comprend en outre :
une unité de changement standard (527) qui est configurée pour changer la valeur standard prédéterminée sur la base d'informations indiquant une partie de mesure pour laquelle l'intensité de courant est calculée.

2. Appareil d'aide au diagnostic selon la revendication 1, l'appareil d'aide au diagnostic comprenant en outre :
une unité de détermination de taux diminuant (526) qui est configurée pour déterminer si le taux diminuant de l'intensité de courant calculée par l'unité de calcul est inférieur ou non à la valeur standard prédéterminée,
dans lequel le contrôleur d'affichage (530) affiche, sur l'unité d'affichage, des informations indiquant un taux diminuant de l'intensité de courant qui est déterminé pour être inférieur à la valeur standard prédéterminée par l'unité de détermination de taux diminuant en tant que résultat de comparaison.

3. Appareil d'aide au diagnostic selon la revendication 2, l'appareil d'aide au diagnostic comprenant en outre :
un analyseur de reconstitution (523) qui reconstitue des composants de courant d'une pluralité de premières électrodes virtuelles installées dans une région prédéterminée incluant une partie où les données de champ magnétique sont acquises sur la base des données de champ magnétique ;
un extracteur de composants de courant (524) qui extrait des composants de courant dans une pluralité de secondes électrodes virtuelles agencées à intervalles prédéterminées le long du trajet nerveux sur une image morphologique d'un sujet, sur la base des composants de courant reconstitués par l'analyseur de reconstitution ; et
un générateur de forme d'onde (532) qui génère une forme d'onde du courant entrant dans la pluralité de secondes électrodes virtuelles, sur la base des composants de courant extraits par l'extracteur de composants de courant,
dans lequel le contrôleur d'affichage (530) associe la forme d'onde de courant générée par le générateur de forme d'onde au résultat de comparaison et amène l'unité d'affichage.

4. Appareil d'aide au diagnostic selon la revendication 3,
dans lequel le contrôleur d'affichage (530) est configuré pour émettre en sortie, vers l'unité d'affichage, des informations représentant le taux diminuant de l'intensité de courant qui est déterminé pour être inférieur à la valeur standard prédéterminée par l'unité de détermination de taux diminuant conjointement avec la forme d'onde de courant correspondante.

5. Appareil d'aide au diagnostic selon la revendication 1,
dans lequel le taux diminuant de l'intensité de courant est exprimé en tant que rapport de CS2/CS1, où une intensité de courant en aval d'une activité nerveuse est représentée par CS2 et une intensité de courant en amont d'une activité nerveuse est représentée par CS1.

6. Appareil d'aide au diagnostic selon l'une quelconque parmi les revendications 1 à 5,
dans lequel l'unité de calcul (520) est configurée pour calculer le taux diminuant de l'intensité de courant, sur la base des données de champ magnétique obtenues en mesurant le champ magnétique généré à partir d'un bas du dos ou d'une région cervicale du sujet.

7. Appareil d'aide au diagnostic selon l'une quelconque des revendications 1 à 5,
dans lequel l'unité de calcul est configurée pour calculer le taux diminuant de l'intensité de courant sur la base des données de champ magnétique obtenues en mesurant le champ magnétique généré dans un nerf périphérique du sujet.

8. Procédé d'aide au diagnostic comprenant :
le calcul d'un taux diminuant d'une intensité de courant d'un courant entrant circulant dans un trajet nerveux, sur la base de données de champ magnétique obtenues en mesurant un champ magnétique généré par un sujet ; et
l'affichage d'un résultat de comparaison dans lequel le taux diminuant de l'intensité de courant est comparé à une valeur standard prédéterminée, sur une unité d'affichage ;
et **caractérisé en ce qu'**il comprend en outre :
le changement de la valeur standard prédéterminée sur la base d'informations indiquant une partie de mesure pour laquelle l'intensité de courant est calculée.

9. Programme d'aide au diagnostic comprenant des instructions de programme configurées pour amener un ordinateur à :
calculer un taux diminuant d'une intensité de courant d'un courant entrant circulant dans un trajet nerveux, sur la base de données de champ magnétique obtenues en mesurant un champ magnétique généré par un sujet ; et
afficher un résultat de comparaison dans lequel le taux diminuant de l'intensité de courant est comparé à une valeur standard prédéterminée, sur une unité d'affichage ;
et **caractérisé en ce qu'**il comprend en outre :
le changement de la valeur standard prédéterminée sur la base d'informations indiquant une partie de mesure pour laquelle l'intensité de courant est calculée.
